# EUROPÄISCHE PATENTSCHRIFT

(11) **EP 0 554 214 B1**
(45) Veröffentlichungstag und Bekanntmachung des Hinweises auf die Patenterteilung: **05.03.1997**
(21) Anmeldenummer: 93810028.6
(22) Anmeldetag: 19.01.1993
(51) Int. Cl.: A61F 2/34

(54) **Verfahren zur Herstellung einer implantierbaren Gelenkschale**
Process for making an implantable acetabular cup
Procédé de fabrication d'une cupule cotyloidienne implantable

(30) Priorität: 31.01.1992 CH 287/92
(43) Veröffentlichungstag der Anmeldung: 04.08.1993
(60) Teilanmeldung: 96106328.6
(73) Patentinhaber: Sulzer Orthopädie AG, 6340 Baar (CH)
(72) Erfinder: Koch, Rudolf, CH-8500 Frauenfeld (CH); Willi, Roland, CH-8413 Neftenbach (CH)
(74) Vertreter: Heubeck, Bernhard

(56) Entgegenhaltungen:
- EP-A- 0 245 527
- EP-A- 0 297 789
- EP-A- 0 445 068
- EP-A- 0 453 694
- GB-A- 2 126 096

## Beschreibung

Die Erfindung handelt von einem Verfahren zur Herstellung einer implantierbaren Gelenkschale, vorzugsweise einer Hüftgelenkschale, die eine äussere metallische Schale, welche zur Verankerung im Knochengewebe dient und eine verschleissfeste Innenschale zur Aufnahme eines Gelenkkopfes aufweist.

Die EP-A-0 453 694 zeigt eine dreiteilige künstliche Hüftgelenkschale, die eine äussere metallische Spreizschale aufweist, welche über ein Hilfswerkzeug in einer Knochenaushöhlung aufspreizbar ist, und die in der aufgespreizten Stellung durch eine Zwischenschale aus Polyethylen mit einem Bajonettverschluss gegen ein Zurückfedern der Verankerungselemente verriegelbar ist. In der Zwischenschale ist eine Innenschale, welche aus einem harten Werkstoff wie beispielsweise Aluminiumoxyd besteht, mit einer Schnappverriegelung verankert. Schnappverriegelungen nutzen die Elastizität der beteiligten Partner aus, um über die Deformation von zueinander geführten Teilen eine definierte Rückhaltekraft zu erzeugen. Dies bedingt sehr genaue und in engen Toleranzen herzustellende Teile, um die gewünschte Verbindung zu erreichen, was für harte und somit schwer zu bearbeitende Innenschalen eine teure Herstellung verursacht.

Zweiteilige Gelenkschalen mit Schnappverriegelungen zwischen Aussen- und Innenschale sind seit vielen Jahren in Gebrauch. Sie erleichtern dem Operateur die Arbeit, indem die Aussenschale ohne Anwesenheit der Innenschale an einem Knochenbett befestigt werden kann und die Innenschale erst später einsetzbar ist. CH-PS 669 904 zeigt eine Anordnung mit einer Kunststoff-Innenschale, die in einer Aussenschale mit einer deformierbaren Schulter einschnappt. Wesentlich schwieriger wird in diesem Fall wie auch allgemein das Befestigungsproblem, wenn für die innerste Schale ein harter, schwer bearbeitbarer und unter Umständen auch spröder Werkstoff verwendet werden soll. Weder Elastizität noch Schlagfestigkeit bestimmter verschleissfester Materialien lassen eine sichere Schnappverbindung oder sichere andere Verbindungen zur Aussenschale zu.

Hier schafft die Erfindung Abhilfe. Sie löst die Aufgabe, Innenschalen aus schwer bearbeitbaren Werkstoffen mit einer Aussenschale zu verbinden. Diese Aufgabe wird mit den kennzeichnenden Merkmalen des unabhängigen Patentanspruchs 1 gelöst.

Die Vorteile der Erfindung sind darin zu sehen, dass der Bearbeitungsaufwand für die Innenschale wesentlich geringer wird, wenn bei gesinterten Werkstoffen, die im ungesinterten Zustand erreichbare Herstellgenauigkeit der Aussenkontur und wenn bei metallischen Werkstoffen, die im Rohling erreichbare Herstellgenauigkeit der Aussenkontur zum Aufbau einer Verbindung zur Aussenschale ausreichend sind. Ein weiterer Vorteil liegt in der einfachen Anpassbarkeit der Innenschale an verschiedene Ausführungsformen von metallischen Aussenschalen, selbst an implantierte Aussenschalen, bei denen die Innenschale ausgewechselt wird.

Bei grossen Stückzahlen lohnt es sich, passende Kunststoffrohlinge in einer Kunststoffspritzform zu erzeugen.

Ein weiterer Vorteil liegt in der Dämpfung des Kunststoffes, der Spannungsspitzen beim Uebertragen von Kräften abbaut. Weitere Vorteile ergeben sich aus den Kennzeichen der abhängigen Ansprüche 2, 3, 4, 5, 6.

Im folgenden wird die Erfindung anhand von Ausführungsbeispielen beschrieben. Es zeigen:
- Figur 1: Schematisch einen Schnitt in der Achse einer verschleissfesten Innenschale, die von einem angespritzten Kunststoffrohling umgeben ist;
- Figur 2: schematisch die Seitenansicht einer verschleissfesten Innenschale mit einer angespritzten und fertig bearbeiteten Kunststoffschale, die über eine Schnappverbindung mit einer äusseren metallischen Schale verbunden ist;
- Figur 3: schematisch die Seitenansicht einer vorgeheizten Innenschale vor dem Zusammenfügen mit einem zugehörigen Kunststoffrohling; und
- Figur 4: schematisch die Seitenansicht der zusammengefügten Teile aus Fig. 3 nach dem Fertigbearbeiten der Kunststoffschale.

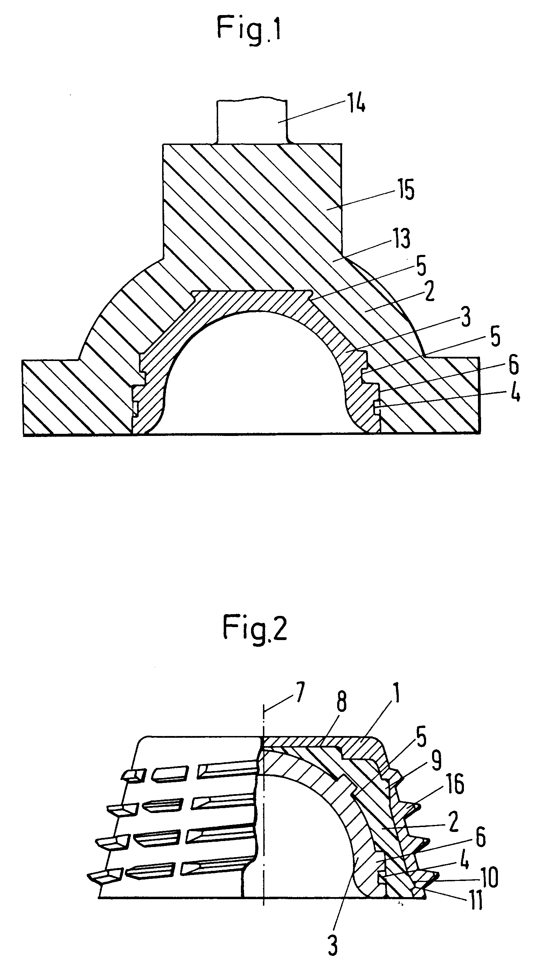

In den Figuren 1 und 2 ist eine verschleissfeste Innenschale 3 und die Entstehung ihrer Verbindung zu einer äusseren metallischen Schale 1 gezeigt. Die verschleissfeste Innenschale 3 wird auf ihrer Innenseite, die später eine Gelenkkugel aufnimmt, fertig bearbeitet, in eine passende Kunststoffspritzform eingelegt und mit einem angespritzten Kunststoffrohling versehen. Dieser ist durch ausgespritzte Vor- und Rücksprünge 4, 5, 6 in der verschleissfesten Innenschale 3 nicht mehr lösbar mit dieser verbunden. Die so geschaffene Verbindung ist für Kräfteübertragung geeignet. Eine so angespritzte Kunststoffschale 2 lässt sich problemlos auf ihre endgültige Form mechanisch bearbeiten und mit der äusseren metallischen Schale lösbar verbinden. Der Kunststoff ist einfach bearbeitbar und hat Dämpfungseigenschaften.

In Figur 1 ist ein Kunststoffrohling 13 auf einer auf der Innenseite fertig bearbeiteten, verschleissfesten Innenschale 3 aufgespritzt. Die Innenschale 3 besitzt auf ihrer umspritzten Aussenseite Vor- und Rücksprünge in Form von Rinnen 5, Bohrungen 4 und Vorsprüngen 6. Bei Innenschalen 3 aus metallischen Werkstoffen genügt es, diese Vor- und Rücksprünge in groben Toleranzen für das Umspritzen vorzubereiten. Bei Innenschalen 3 aus Sintergefüge, wie z.B. Metalloxiden genügt es von der Genauigkeit her die Vor- und Rücksprünge im ungesinterten Zustand zu erzeugen. Die nicht zu umspritzende Innenseite der Innenschale wird vor dem Umspritzen fertig bearbeitet. Anschliessend wird die Innenschale 3 in eine Kunststoffspritzform (nicht gezeigt) eingelegt und umspritzt. Figur 1 entspricht einem entformten Teil, das an seinem Anguss 14 abgetrennt wurde. Der Anguss 14 geht zunächst in ein Reservevolumen 15 über, aus dem flüssiges Material nachgesogen werden kann, wenn der Kunststoff auf der Innenschale 3 abkaltet. Der Kunststoffrohling 13 ist mit reichlicher Zugabe versehen, um durch mechanische Bearbeitung des Kunststoffs die Passungen zu verschiedenen Aussenschalen 1 in Form von unterschiedlich dimensionierten Kunststoffschalen 2 herzustellen.

Bei grossen Stückzahlen von gleichen Aussenschalen 1 ist es denkbar, bis auf den Anguss die Endform der Kunststoffschale 2 mit dem Umspritzen herzustellen. Im Beispiel der Figur 2, die eine Schnappverbindung 11 mit Aufnahmefläche 10 zu einer selbstschneidenden äusseren metallischen Schale 1 zeigt, müsste die Formtrennung auf Höhe der Nase der Schnappverbindung 11 verlaufen, damit Aufnahmeflächen wie die Aufnahmeflächen 8, 9, 10 mit dem Spritzvorgang erzeugt werden.

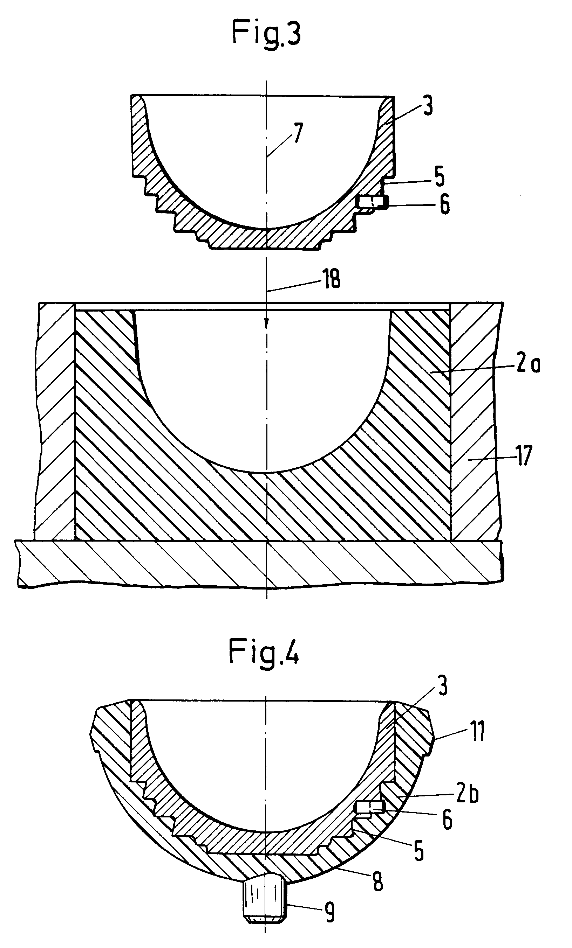

In Figur 3 ist eine verschleissfeste Innenschale 3 mit hinterschnittenen Rücksprüngen 5 auf der Aussenseite gezeigt. Ein eingepresster Stift steht als Vorsprung 6 vor, welcher später als Verdrehsicherung wie in Figur 4 wirkt. Ein Kunststoffrohling 2a mit zylindrischer Aussenform ist in einem Stützring 17 gefangen und besitzt eine Innenwölbung, welche der Hüllkurve der Aussenfläche der Innenschale 3 entspricht. Die Innenschale 3 wird in einer Einpressrichtung 18 in den Kunststoffrohling 2a eingepresst. Während oder kurz vor dem Einpressen wird die Innenwölbung angeschmolzen, um flüssigen Kunststoff in die Hinterschneidungen 5 fliessen zu lassen. Das Anschmelzen der Innenwölbung kann durch vorheriges Aufheizen der Innenschale 3 geschehen oder/und durch kurzzeitiges Aufheizen der Innenwölbung mit z.B. einem Heissluftstrom aus einer Düse (nicht gezeigt). Nach dem Einpressen der Innenschale 3 und dem Abkühlen des Kunststoffs ist eine dauerhafte Verbindung entstanden, die bei einer Aufnahme an der Innenschale 3 eine spanende Formgebung am Kunststoffrohling 2a ermöglicht.

Figur 4 und 2 zeigen eine Innenschale 3 mit fertig bearbeiteter Kunststoffschale 2b. Schnappverbindungen 11, Aufnahmeflächen 8, 9, 10 und Anschläge können durch die spanende Formgebung jeweils einer metallischen äusseren Schale 1 angepasst werden.

Figur 2 zeigt weiterhin eine Aussenschale 1 mit selbstschneidendem Gewinde 16 und eine Innenschale 3 mit Kunststoffschale 2, deren Kontaktflächen rotationssymmetrisch zur Polachse 7 liegen und jeweils in einer Aufspannung durch Drehen erzeugt werden können.

Die Wahl der Werkstoffe für die Innenschale 3 ist damit viel weniger von den Bearbeitungskosten beeinflusst und es kommen dafür auch schwer bearbeitende Kobalt-Chrom-Molybdänlegierungen, Kobalt-Chrom-Kohlenstofflegierungen und Sintergefüge wie z.B. aus Metalloxiden in Frage. Für die Lagerhaltung ergeben sich weitere Vorteile, indem bei einem Zwischenlager von mit Kunststoffrohlingen 2a versehenen verschleissfesten Innenschalen 3 unterschiedliche Aussenformen wegen der einfachen Bearbeitbarkeit als Fertigteile schnell verfügbar sind. Die Verbindung zwischen der äusseren metallischen Schale 1 und der Innenschale 3 mit Kunststoffschale 2b kann je nach Anforderung vorgenommen werden. Eine werkstattmässige Montage ist ebenso möglich, wie das Einsetzen einer Innenschale 3 mit Kunststoffschale 2b in eine bereits implantierte äussere metallische Schale. Innenschalen 3 mit Kunststoffschalen 2b können auf diese Weise auch als Ersatz für weniger verschleissfeste Innenschalen in bereits eingewachsene Aussenschalen eingesetzt werden.

## Patentansprüche

1. Verfahren zur Herstellung einer implantierbaren Gelenkschale, vorzugsweise einer Hüftgelenkschale, die eine äussere metallische Schale (1), welche zur Verankerung im Knochengewebe dient, und eine verschleissfeste Innenschale (3) zur Aufnahme eines Gelenkkopfes aufweist, wobei
- in einem ersten Schritt die verschleissfeste Innenschale (3), auf deren Aussenseite zu ihrer Polachse (7) radiale Vor- und Rücksprünge (4, 5, 6) angebracht sind, mechanisch fertig bearbeitet wird; und
- in einem weiteren Schritt eine mittlere Kunststoffschale (2b) mit der äusseren metallischen Schale (1) verbunden wird,
dadurch gekennzeichnet, dass
- in einem zweiten Schritt die verschleissfeste Innenschale (3) durch Schmelzfliessen mit einem Kunststoffrohling (2a) verbunden wird, wobei über in die Rücksprünge (4, 5) einfliessendes erschmolzenes Kunststoffmaterial nach einer Abkühlung eine dauerhafte nicht mehr lösbare Verbindung zwischen verschleissfester Innenschale (3) und Kunststoffrohling (2a) entsteht.
- in einem dritten Schritt der Rohling (2a) zu einer mittleren Kunststoffschale (2b) mit Aufnahmeflächen (8, 9, 10) für das Anbringen der äusseren metallischen Schale (1) nachgeformt wird, und
- in einem vierten Schritt, die mittlere Kunstoffschale zusammen mit der Innenschale (3) über die Aufnahmeflächen (8, 9, 10) mit der äusseren metallischen Schale (1) verbunden wird.

2. Verfahren nach Anspruch 1, dadurch gekennzeichnet, daß im dritten Schritt eine Schnappverbindung (11) nachgearbeitet wird, um die mittlere Kunststoffschale (2b) mit der äusseren metallischen Schale (1) zu verbinden.

3. Verfahren nach Anspruch 1, dadurch gekennzeichnet, dass im zweiten Verfahrensschnitt die verschleissfeste Innenschale (3) in eine Kunststoffspritzform eingelegt wird und eine mittlere Kunststoffschale (2b) als Rohling (2a) an die verschleissfeste Innenschale (3) angespritzt wird, wobei über die ausgespritzten Rücksprünge (4, 5, 6) eine nicht mehr lösbare, zur Kräfteübertragung geeignete Verbindung zwischen den Schalen (2, 3) besteht.

4. Verfahren nach Anspruch 1, dadurch gekennzeichnet, dass im zweiten Verfahrensschritt die Innenwölbung von einem Kunststoffrohling (2a) angeschmolzen wird und die Innenschale eingepresst wird, um eine dauerhafte Verbindung zu erzeugen.

5. Verfahren nach Anspruch 1, dadurch gekennzeichnet, dass im zweiten Verfahrensschnitt die Innenschale (3) vorgeheizt und in einen Kunststoffrohling (2a) eingepresst wird, wobei das lokal erschmolzene Kunststoffmaterial in die Rücksprünge (4, 5) der Innenschale einfliesst, um nach Abkühlung eine nicht mehr lösbare, zur Kräfteübertragung geeignete Verbindung zwischen Innenschale (3) und Kunststoffrohling (2a) zu erzeugen.

6. Verfahren nach einem der Ansprüche 1 bis 5, dadurch gekennzeichnet, dass es auf Innenschalen (3) angewendet wird, welche aus einer metallischen Legierung wie z.B. aus einer Kobalt-Chrom-Molybdänlegierung oder einer Kobalt-Chrom-Kohlenstofflegierung bestehen, oder welche aus einem nicht-metallischen, schwer zu bearbeitenden Werkstoff wie z.B. aus gesinterten Metalloxiden bestehen.

## Claims

1. A process for the manufacture of an implantable articular cup, preferably a hip joint cup, which comprises an outer metallic cup (1), which is used for attachment in the osseous tissue, and a wear-resistant inner cup (3) for receiving a condyle,
whereby
- in a first step the wear-resistant inner cup (3), on the outside of which radial projections and recesses (4, 5, 6) to its polar axis (7) are mounted, is finished by machine,
- in a further step a central plastic cup (2b) is connected to the outer metallic cup (1),
**characterised in that**
- in a second step the wear-resistant inner cup (3) is connected to a plastic blank (2a) by fusing, whereby a permanent, no longer detachable connection is produced between the wear-resistant inner cup (3) and plastic blank (2a) via molten plastic material flowing into the recesses (4, 5) after cooling,
- in a third step the blank (2a) is postformed to form a central plastic cup (2b) with receiving surfaces (8, 9, 10) for the mounting of the outer metallic cup (1), and
- in a fourth step, the central plastic cup together with the inner cup (3) is connected via the receiving faces (8, 9, 10) to the outer metallic cup (1).

2. A process according to Claim 1,
**characterised in that** in the third step a snap connection (11) is subsequently worked in order to connect the central plastic cup (2b) with the outer metallic cup (1).

3. A process according to Claim 1,
**characterised in that** in the second process step the wear-resistant inner cup (3) is inserted into a plastic injection mould and a central plastic cup (2b) is injected as a blank (2a) on to the wear-resistant inner cup (3), whereby a connection which is no longer detachable and is suitable for the transmission of forces exists between the cups (2, 3) via the injected recesses (4, 5, 6).

4. A process according to Claim 1,
**characterised in that** in the second process step the inner curvature of a plastic blank (2a) is melted and the inner cup is pressed in to produce a permanent connection.

5. A process according to Claim 1,
**characterised in that** in the second process step the inner cup (3) is preheated and pressed into a plastic blank (2a), whereby the locally molten plastic material flows into the recesses (4, 5) in the inner cup so as, after cooling, to produce a connection which is no longer detachable and is suitable for the transmission of forces between inner cup (3) and plastic blank (2a).

6. A process according to one of Claims 1 to 5,
**characterised in that** it is used on inner cups (3), which are made from a metallic alloy such as, for example, a cobalt-chromium-molybdenum alloy or a cobalt-chromium-carbon alloy, or which are made from a non-metallic, difficult-to-work material, such as e.g. sintered metal oxides.

## Revendications

1. Procédé de fabrication d'une cupule cotyloïdienne implantable, de préférence une cupule acétabulaire, présentant une coquille métallique extérieure (1), servant à l'ancrage dans le tissu osseux, et une coquille intérieure (3) résistant à l'usure, servant à recevoir une tête d'articulation, dans lequel :
- lors d'une première étape, la coquille intérieure (3) résistant à l'usure, sur la face extérieure de laquelle sont ménagés des saillies et des retraits (4, 5, 6) radiaux par rapport à son axe polaire (7), est soumise à un usinage mécanique de finition; et
- lors d'une étape supplémentaire, une coquille en matière synthétique (2b) médiane est reliée à la coquille métallique extérieure (1),
caractérisé en ce que
- lors d'une deuxième étape, la coquille intérieure (3) résistant à l'usure est reliée, par coulée d'un produit en fusion, à une ébauche en matière synthétique (2a), le matériau synthétique fondu s'étant écoulé dans les retraits (4, 5) établissant, après refroidissement, une liaison permanente, qui n'est plus désolidarisable, entre la coquille intérieure (3) résistant à l'usure et l'ébauche en matière synthétique (2a).
- lors d'une troisième étape, l'ébauche (2a) est refaçonnée pour donner une coquille en matière synthétique médiane (2b) et dotée de surfaces-supports (8, 9, 10) destinées au montage de la coquille extérieure (1), et
- dans une quatrième étape, la coquille en matière synthétique médiane est reliée, conjointement avec la coquille intérieure (3), par l'intermédiaire des surfaces-supports (8, 9, 10), à la coquille métallique extérieure (1).

2. Procédé selon la revendication 1, caractérisé en ce que, dans une troisième étape, une liaison à encliquetage (11) est retravaillée, pour relier la coquille en matière synthétique médiane (2b) à la coquille métallique extérieure (1).

3. Procédé selon la revendication 1, caractérisé en ce que, dans la deuxième étape du procédé, la coquille intérieure (3) résistant à l'usure est insérée dans un moule d'injection de matière synthétique et une coquille en matière synthétique médiane (2b) est surmoulée par injection, à titre d'ébauche (2a), sur la coquille intérieure (3) résistant à l'usure, une liaison devenue impossible à désolidariser, convenant pour la transmission d'efforts, étant constituée entre les coquilles (2, 3), par l'intermédiaire des retraits (4, 5, 6) venant du moulage par injection.

4. Procédé selon la revendication 1, caractérisé en ce que, dans la deuxième étape du procédé, la courbure intérieure d'une ébauche en matière synthétique (2a) est portée à fusion et la coquille intérieure est insérée avec enfoncement pour produire une liaison permanente.

5. Procédé selon la revendication 1, caractérisé en ce que, dans la deuxième étape du procédé, la coquille intérieure (3) est préchauffée et enfoncée dans une ébauche en matière synthétique (2a), le matériau synthétique fondu localement pénétrant à l'état liquide dans les retrait (4, 5) de la coquille intérieure afin, après refroidissement, de produire une liaison ne pouvant plus être désolidarisée, convenant pour la transmission d'efforts, entre la coquille intérieure (3) et l'ébauche en matière synthétique (2a).

6. Procédé selon l'une des revendications 1 à 5, caractérisé en ce qu'il est appliqué à des coquille intérieures (3) qui sont constituées d'un alliage métallique, tel que, par exemple, un alliage cobalt-chrome-molybdène ou un alliage cobalt-chrome-carbone, ou qui sont constituées d'un matériau non métallique, difficile à usiner, tel que, par exemple, des oxydes métalliques frittés.
